# EUROPÄISCHE PATENTSCHRIFT

(11) **EP 0 733 612 B1**
(45) Veröffentlichungstag und Bekanntmachung des Hinweises auf die Patenterteilung: **08.09.1999**
(21) Anmeldenummer: 96103773.6
(22) Anmeldetag: 11.03.1996
(51) Int. Cl.: C07C 17/389, C07C 19/08

(54) **Verfahren zur Entfernung vom olefinischen Verunreinigungen aus 2H-Heptafluorpropan (R 227)**
Process for removing olefinic impurities from 2H-heptafluoropropane (R 227)
Procédé d'élimination d'impurités oléfiniques de 2H-heptafluoropropane (R 227)

(30) Priorität: 21.03.1995 DE 19510159
(43) Veröffentlichungstag der Anmeldung: 25.09.1996
(73) Patentinhaber: SOLVAY (Société Anonyme), 1050 Bruxelles (BE)
(72) Erfinder: Jansen, Rolf-Michael, Dr., D-65835 Liederbach (DE); Hopp, Peter, Dr., D-65719 Hofheim (DE)
(74) Vertreter: Jacques, Philippe

(56) Entgegenhaltungen:
- EP-A- 0 512 502
- US-A- 3 026 359
- US-A- 3 696 156

## Beschreibung

Der nur teilweise fluorierte, chlorfreie Kohlenwasserstoff (H-FKW) 2H-Heptafluorpropan (R 227) ist ein sehr geeignetes Substitut für die bisher in Pharma-Aerosolen als Treibmittel verwendeten FCKW. Bei der Herstellung von R 227 aus Hexafluorpropen (HFP) kann jedoch die Bildung von toxischen olefinischen Nebenprodukten nicht völlig verhindert werden. Ebenso kann nicht verhindert werden, daß das olefinische Ausgangsprodukt HFP in Spuren im R 227 vorhanden ist. Für die Anwendung von R 227 als Pharma-Aerosol müssen diese olefinischen Verunreinigungen, auch wenn sie nur im ppm-Bereich auftreten, vollständig entfernt werden.

Es ist bekannt, daß sich olefinische Verunreinigungen aus R 227 beispielsweise mit Alkoholen und einer Base entfernen lassen (EP-A-0 512 502). Man hat dabei aber das Problem, daß die unumgesetzten Reste des Alkohols anschließend selbst wieder vollständig aus dem R 227 entfernt werden müssen. Außerdem müssen die bei dieser Reaktion entstehenden fluorierten Ether aufwendig beseitigt werden.

Aus US-A-3 696 156 ist die Entfernung von Perfluorolefinen oder Perfluorchlorolefinen aus gesättigten perhalogenierten Fluorkohlenwasserstoffen mit 2 bis 6 C-Atomen in der Gasphase bei relativ hohen Temperaturen von 180 bis 250°C bekannt. Dabei wird das gasförmige Ausgangsmaterial über Aluminiumoxid geleitet, das eine basische Verbindung, wie ein Alkalimetall-Hydroxid oder -Oxid enthält. Die genannten Temperaturen sollten nicht unterschritten werden (Spalte 4, Zeile 12 bis 19). Ebenso ist die Gegenwart der basischen Verbindungen auf dem Aluminiumoxid notwendig (Spalte 3, Zeile 52 bis 66).

Nach EP-A-0 457 613 kann man die flüssigen höheren Homologen der genannten perhalogenierten Verbindungen bereits bei Raumtemperatur analog reinigen, beispielsweise flüssige Perfluoralkane mit 5 bis 8 C-Atomen. Dabei wird wieder vorzugsweise Aluminiumoxid eingesetzt, das mit Alkali- oder Erdalkalimetall-Hydroxid oder -Oxid beschichtet worden ist (Seite 3, Zeile 9 bis 21).

Es hat sich nun überraschenderweise gezeigt, daß sich olefinische Verunreinigungen, insbesondere Hexafluorpropen (HFP), schon bei -20 bis 100°C in der Gasphase vollständig aus R 227 entfernen lassen, wenn man das verunreinigte R 227 über Aluminiumoxid leitet.

Die Erfindung betrifft somit ein Verfahren zur Entfernung olefinischer Verunreinigungen aus 2H-Heptafluorpropan (R 227), dadurch gekennzeichnet, daß man das verunreinigte R 227 in der Gasphase bei -20 bis 100°C über Aluminiumoxid leitet. Vorzugsweise enthält das Aluminiumoxid 2 bis 10 Gew.-% adsorbiertes Wasser, insbesondere 2 bis 8 Gew.-%.

Vorzugsweise arbeitet man bei einer Temperatur von -20 bis 50°C, insbesondere von 0 bis 30°C. Der Druck beträgt vorzugsweise 1 bis 10 bar.

Vorzugsweise wird γ-Aluminiumoxid eingesetzt.

Das Aluminiumoxid braucht im Unterschied zu den in US-A-3 696 156 sowie EP-A-0 457 613 beschriebenen Verfahren keine basischen Verbindungen wie Alkalimetalloxide oder -hydroxide, oder Erdalkalimetalloxide oder -hydroxide zu enthalten. Vorzugsweise ist das Aluminiumoxid frei von solchen Verbindungen, da diese beim vorliegenden Verfahren überflüssig sind, obwohl sie nicht schaden.

Die Erfindung soll anhand des folgenden Beispiels näher erläutert werden.

### Beispiel

400 kg R 227 mit einem Gehalt von 50 ppm Hexafluorpropen wurden im Kreislauf aus einem Vorratsbehälter bei einem Druck von 5 bar mit einer Pumpe (Durchsatz 50 kg/h) bei 25°C von unten durch ein Rohr (Länge = 100 cm, Durchmesser = 10 cm) geleitet, das mit 10 kg Aluminiumoxid (Wassergehalt 6 Gew.-%) gefüllt war und dann wieder in den Vorratsbehälter zurückgeführt. Nachdem das R 227 24 Stunden lang im Kreislauf über das Aluminiumoxid geleitet worden war, lag der Hexafluorpropen-Gehalt im R 227 unterhalb der Nachweisgrenze von 1 ppm.

## Patentansprüche

1. Verfahren zur Entfernung olefinischer Verunreinigungen aus 2H-Heptafluorpropan (R 227), dadurch gekennzeichnet, daß man das verunreinigte R 227 in der Gasphase bei -20 bis 100°C über Aluminiumoxid leitet.

2. Verfahren nach Anspruch 1, dadurch gekennzeichnet, daß man bei einer Temperatur von -20 bis 50°C arbeitet.

3. Verfahren nach Anspruch 1 oder 2, dadurch gekennzeichnet, daß man bei einem Druck von 1 bis 10 bar arbeitet.

4. Verfahren nach einem der Ansprüche 1 bis 3, dadurch gekennzeichnet, daß man Aluminiumoxid in der γ-Modifikation verwendet.

5. Verfahren nach einem der Ansprüche 1 bis 4, dadurch gekennzeichnet, daß das Aluminiumoxid 2 bis 10 Gew.-% adsorbiertes Wasser enthält.

## Claims

1. A process for removing olefinic impurities from 2H-heptafluoropropane (R 227), characterised in that the contaminated R 227 is passed in the gas phase at from -20 to 100 °C over aluminum oxide.

2. The process as claimed in claim 1, characterised in that it is carried out at a temperature of from -20 to 50 °C.

3. The process as claimed in claim 1 or 2, characterised in that it is carried out at a pressure of from 1 to 10 bar.

4. The process as claimed in any of claims 1 to 3, characterised in that the aluminum oxide used is in the γ-modification.

5. The process as claimed in any of claims 1 to 4, characterised in that the aluminum oxide contains from 2 to 10% by weight of adsorbed water.

## Revendications

1. Procédé pour éliminer des impuretés oléfiniques du 2H-heptafluoropropane (R 227), caractérisé en ce qu'on guide le R 227 pollué en phase gazeuse à une température de -20 à 100°C par-dessus de l'oxyde d'aluminium.

2. Procédé selon la revendication 1, caractérisé en ce qu'on travaille à une température de -20 à 50°C.

3. Procédé selon la revendication 1 ou 2, caractérisé en ce qu'on travaille sous une pression de 1 à 10 bar.

4. Procédé selon l'une quelconque des revendications 1 à 3, caractérisé en ce qu'on utilise de l'oxyde d'aluminium dans la modification γ.

5. Procédé selon l'une quelconque des revendications 1 à 4, caractérisé en ce que l'oxyde d'aluminium contient de l'eau à l'état adsorbé à concurrence de 2 à 10% en poids.
